# EUROPÄISCHE PATENTSCHRIFT

(11) **EP 1 299 367 B1**
(45) Veröffentlichungstag und Bekanntmachung des Hinweises auf die Patenterteilung: **26.11.2003**
(21) Anmeldenummer: 01965255.1
(22) Anmeldetag: 05.09.2001
(51) Int. Cl.: C07D 301/03

(54) **VERFAHREN ZUR HERSTELLUNG VON EPOXIDEN DURCH OXIDATION VON OLEFINEN**
METHOD FOR PRODUCING EPOXIDES BY OXIDISING OLEFINS
PROCEDE DE FABRICATION D'EPOXYDES PAR OXYDATION D'OLEFINES

(30) Priorität: 05.09.2000 DE 10044538
(43) Veröffentlichungstag der Anmeldung: 09.04.2003
(73) Patentinhaber: I.F.T. Institut für Troposphärenforschung e.V., 04303 Leipzig (DE)
(72) Erfinder: BERNDT, Torsten, 37327 Leinefelde (DE); BÖGE, Olaf, 04509 Schkeuditz OT Glesien (DE); HEINTZENBERG, Jost, 04107 Leipzig (DE)
(74) Vertreter: Ziebig, Marlene, Dr. Dipl.-Chem.
(86) Internationale Anmeldenummer: EP0110231
(87) Internationale Veröffentlichungsnummer: WO02020502

(56) Entgegenhaltungen:
- WO-A-97/28142
- WO-A-97/34693
- WO-A-99/29679
- DE-B- 1 219 014
- US-A- 5 760 254

## Beschreibung

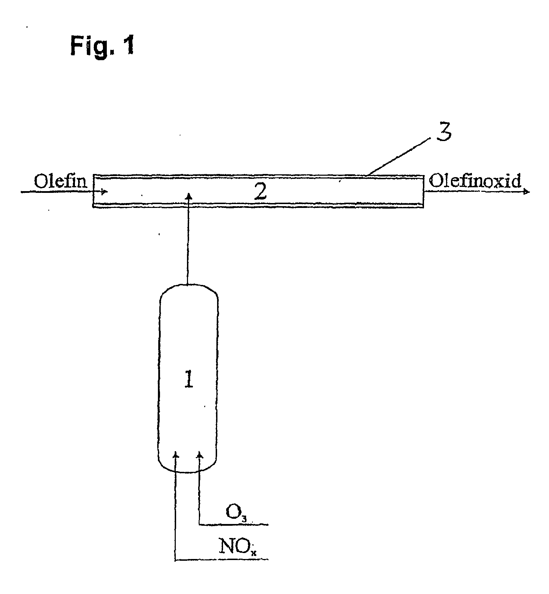

Die Erfindung betrifft ein neues Verfahren zur Herstellung von Epoxiden durch Oxidation von Olefinen in einer homogenen Gasphasenreaktion, indem Ozon und NO₂ und/oder NO bei milden Reaktionsbedingungen und ohne Verwendung eines Katalysators mit dem gewünschten olefin umgesetzt werden. Das erfindungsgemäße Verfahren kann als kontinuierliches Einstufen-Verfahren im Reaktor gemäß Figur 1 durchgeführt werden und erfordert einen sehr geringen technischen Aufwand. Es können Monoolefine mit 2 bis 16 Kohlenstoffatomen und Diolefine mit 4 bis 16 Kohlenstoffatomen epoxidiert werden.

Epoxide sind wichtige Zwischenprodukte der chemischen Industrie, die hauptsächlich zur Herstellung von Olefinglycolen bzw. deren Di-, Poly- oder Oligomeren dienen, die vorwiegend zu Polyurethanen weiterverarbeitet werden. Insbesondere Propylenoxid und Ethylenoxid werden jährlich jeweils ca. 5 Mio. Tonnen benötigt.

Epoxide können aus Olefinen durch das Chlorhydrinverfahren, durch indirekte Oxidationsverfahren mittels Peroxid-Reagentien und durch katalytische oder nicht-katalytische Direktoxidationsverfahren hergestellt werden. Die Oxidationen können in der Flüssigphase oder in der Gasphase durchgeführt werden. Oxidationen in der Flüssigphase, die homogen oder heterogen durchgeführt werden können, sind mit schwierigen Trennaufgaben und komplizierten Technologien verbunden. Direktoxidationsverfahren von Olefinen in der Gasphase weisen relativ lange Verweilzeiten auf und liefern meist zu geringe Umsätze und/oder zu geringe Selektivitäten an Epoxid.

So beschreibt beispielsweise DE 197 54 303 A1 ein Verfahren zur Herstellung von Propylenoxid aus Propylen in einer homogenen Gasphasenreaktion, das zwar eine Selektivität zu Propylenoxid > 60% aufweist, allerdings sind die Propylenumsätze mit 13% bzw. 15% relativ gering. Das Verfahren ist daneben technisch aufwendig und teuer, da der Reaktorinnenraum erfindungsgemäß mit inertem Material, insbesondere Edelmetallen, ausgekleidet ist.

Aufgabe der vorliegenden Erfindung war es, ein Verfahren zur Herstellung von Epoxiden durch Oxidation von Olefinen bereitzustellen, das einen geringen technischen Aufwand erfordert, kostengünstig ist und sowohl hohe Selektivitäten an erzeugtem Epoxid als auch hohe Umsätze an eingesetztem Olefin gewährleistet.

Es wurde gefunden, dass Epoxide durch Oxidation von Olefinen in einem homogenen, kontinuierlichen Gasphasenverfahren mit guten Umsätzen und guten Selektivitäten an erzeugtem Epoxid hergestellt werden können, indem man Ozon und NO₂ und/oder NO, ggf. unter Verwendung eines Trägergases, mischt, die resultierende Gasmischung einem üblichen Strömungsreaktor zuführt und in diesem mit dem entsprechenden Olefin, das mittels Trägergas zugeführt wird, bei milden Reaktionsbedingungen umsetzt. Erfindungsgemäß ist lediglich ein Druck von 0,1 - 1000 mbar, vorzugsweise von 1 - 500 mbar, besonders bevorzugt von 1 - 200 mbar, erforderlich. Die notwendigen Temperaturen betragen von 50 - 350°C, vorzugsweise von 100 - 300°C, besonders bevorzugt von 140 - 240°C. Das erfindungsgemäße Verfahren erfordert keinen Katalysator. Die Verweilzeiten in der Reaktionszone betragen von 0,1 ms bis maximal einige wenige Sekunden. Vorzugsweise liegen sie zwischen 0,1 und 300 ms.

Als Trägergase können Sauerstoff sowie Inertgase wie Helium, Argon, Stickstoff oder deren Mischungen mit Sauerstoff Verwendung finden.

Erfindungsgemäß bevorzugt werden Ozon und NO₂ in einem Verhältnis kleiner 0,5 eingesetzt. Ozon und NO werden vorzugsweise in einem Verhältnis kleiner 1,5 eingesetzt.

In einer bevorzugten Ausführungsform der Erfindung wird Ozon als Ozon/Sauerstoffgemisch zugeführt, vorzugsweise 1-15 Vol% Ozon in Sauerstoff, ganz besonders bevorzugt 5-10 Vol% Ozon in Sauerstoff.

Das erfindungsgemäße Verfahren wird in einem üblichen Strömungsreaktor durchgeführt, dem die Gasmischung aus Ozon und NO₂ und/oder NO und ggf. Trägergas zugeführt wird. Vorzugsweise erfolgt die Durchführung in einem Reaktor gemäß Figur 1.

In Figur 1 bedeuten:
- 1: Mischkammer
- 2: Strömungsreaktor
- 3: beheizbarer Außenmantel

In der Mischkammer 1, die mit dem Strömungsreaktor 2 verbunden ist, werden Ozon und NO₂ und/oder NO, ggf. mit dem Trägergas, vermischt. Dies kann vorzugsweise bei Raumtemperatur geschehen. Der Strömungsreaktor ist aus den üblichen, unter Druckverhältnissen stabilen Materialien gefertigt. Es ist nicht erforderlich, dass er mit inerten Materialien beschichtet ist . Auch in einem Strömungsreaktor aus normalem Standardstahl werden die erfindungsgemäßen Ergebnisse erzielt. Gegebenenfalls kann an den Strömungsreaktor ein GC-MS-Analysator und/oder eine Gasdurchflusszelle mit FT-IR-Spektrometer angeschlossen sein, je nach beabsichtigter Analyse des Reaktionsgases.

Das erfindungsgemäße Verfahren ist ein universell anwendbares Verfahren, indem es Epoxidierungen von Monoolefinen mit 2 bis 16 Kohlenstoffatomen, vorzugsweise mit bis zu 5 Kohlenstoffatomen, ganz besonders bevorzugt von Propylen und den C4-Olefinen, und von Diolefinen mit 4 bis 16 Kohlenstoffatomen erlaubt. Der Reaktor gemäß Figur 1 ist für alle angegebenen Olefine einsetzbar.

Erfindungsgemäß wird somit ein kontinuierliches Einstufen-Verfahren zur Verfügung gestellt, das einen sehr geringen technischen Aufwand erfordert, unter milden Reaktionsbedingungen arbeitet, keinen Katalysator benötigt und sehr geringe Verweilzeiten in der Reaktionszone, also große Durchsätze, aufweist. Die Selektivitäten an erzeugtem Epoxid liegen bei mindestens 68%, wobei daneben hohe Umsätze von mindestens ca. 50% erreicht werden.

Nachfolgend soll die Erfindung an Ausführungsbeispielen näher erläutert werden.

### Ausführungsbeispiele

### Beispiel 1: Oxidation von Propylen zu Propylenoxid

Es wird ein Strömungsreaktor 2 mit Mischkammer 1 gemäß Fig. 1 eingesetzt, wobei die Länge des Strömungsreaktors 60 cm (Reaktionslänge 35 cm) und der Innendurchmesser 16 mm beträgt. Der Strömungsreaktor besteht aus Quarz und ist mit einem heizbaren Außenmantel ausgestattet. Als Trägergas für das Olefin wird He verwendet. In der Mischkammer werden 11,5 Vol.% Ozon mit ca. 23 Vol.% NO₂ sowie 65,5 VoI.% Sauerstoff zusammengebracht. Alle Reaktanten werden über Massenflußregler gasförmig dosiert. Es wird kein Katalysator verwendet.
a) Es wird bei einem Druck von 25 mbar und einer Temperatur von 180°C umgesetzt.
b) Es wird bei einem Druck von 10 mbar und einer Temperatur von 140°C umgesetzt.

Die Ergebnisse der Beispiele 1a und 1b sind in Tabelle 1 zusammengefasst:

| Beispiel | Druck [mbar] | Temperatur [C] | Verweilzeit [s] | Propylenanteil [Vol.%] | Propylenumsatz [Mol %] | Selektivität [Mol %] |
|---|---|---|---|---|---|---|
| 1a | 25 | 180 | 0.25 | 1.9 | 49.6 | 68.9 |
| 1b | 10 | 140 | 0.28 | 4.0 | 52.2 | 81.3 |

In der Tabelle bedeuten:
- Verweilzeit =: Verweilzeit des Gasgemisches in der Reaktionszone des Strömungsreaktors
- Propylenanteil = in Vol.%: am Gesamtgasstrom in der Reaktionszone
- Propylenumsatz = [Mol %]: Verhältnis von umgesetzten Molen an Propylen zu eingesetzten Molen an Propylen mal 100%
- Selektivität = [Mol%]: Verhältnis von gebildeten Molen an Propylenoxid zu umgesetzten Molen an Propylen mal 100%

Es zeigt sich, dass mit dem erfindungsgemäßen Verfahren ohne Verwendung eines Katalysators und trotz milder Reaktionsbedingungen äußerst geringe Verweilzeiten in der Reaktionszone und damit große Durchsätze (Raum-Zeit-Ausbeuten) möglich sind, wobei die Selektivität an Propylenoxid sehr hoch ist.

### Beispiel 2: oxidation von trans-Butylen zu cis/trans-Butylenoxid (Isomerengemisch)

Es wird unter den gleichen Bedingungen wie in Beispiel 1 umgesetzt.
a) Die Umsetzung erfolgt bei einem Druck von 25 mbar und einer Temperatur von 180 °C.
b) Die Umsetzung erfolgt bei einem Druck von 25 mbar und einer Temperatur von 230 °C.

Die Ergebnisse der Beispiele 2a und 2b sind in Tabelle 2 zusammengefasst:

| Beispiel | Druck [mbar] | Temperatur [C] | Verweilzeit [s] | Butylenanteil [Vol.%] | Butylenumsatz[Mol %] | Selektivität [Mol %] |
|---|---|---|---|---|---|---|
| 2a | 25 | 180 | 0.25 | 1.45 | 84.3 | 80.0 |
| 2b | 25 | 230 | 0.23 | 1.45 | 53.1 | 96.9 |

Die Bedeutungen in Tabelle 2 entsprechen denen in Tabelle 1.

Die Ergebnisse zeigen auch hier, dass hohe Umsätze an eingesetztem Olefin und große Durchsätze erzielt werden. Die Selektivität an *cis*/*trans*-Butylenoxid beträgt nahezu 100%.

### Beispiel 3: Oxidation von iso-Butylen zu iso-Butylenoxid

Es wird unter den Bedingungen wie in Beispiel 1 bei einem Druck von 10 mbar und einer Temperatur von 230°C umgesetzt. Die Reaktionslänge beträgt 12 cm. Die Ergebnisse sind in Tabelle 3 dargestellt.

| Beispiel | Druck [mbar] | Temperatur [°C] | Verweilzeit [s] | iso-Butylenanteil [Vol.%] | iso-Butylenumsatz [Mol %] | Selektivität [Mol %] |
|---|---|---|---|---|---|---|
| 3 | 10 | 230 | 0.078 | 3,5 | 74,9 | 75.2 |

Es werden hohe Umsätze an eingesetztem Olefin und eine hohe Selektivität erzielt.

## Patentansprüche

1. Verfahren zur Herstellung von Epoxiden durch Oxidation von Olefinen in einer homogenen Gasphasenreaktion,
**dadurch gekennzeichnet, dass**
Ozon und NO₂ und/oder NO gemischt werden, die resultierende Gasmischung einem Strömungsreaktor zugeführt und in diesem mit dem Olefin bei einem Druck von 0,1 - 1000 mbar und einer Temperatur von 50 - 350°C umgesetzt wird.

2. Verfahren nach Anspruch 1,
**dadurch gekennzeichnet, dass**
bei einer Temperatur von 100 - 300°C, vorzugsweise von 140 - 240°C, umgesetzt wird.

3. Verfahren nach Anspruch 1,
**dadurch gekennzeichnet, dass**
bei einem Druck von 1 - 500 mbar, vorzugsweise 1 - 200 mbar, umgesetzt wird.

4. Verfahren nach Anspruch 1,
**dadurch gekennzeichnet, dass**
Monoolefine eingesetzt werden, die 2 bis 16 Kohlenstoffatome aufweisen oder Diolefine mit 4 bis 16 Kohlenstoffatomen.

5. Verfahren nach Anspruch 1,
**dadurch gekennzeichnet, dass**
Ozon und NO₂ und/oder NO in einer mit dem Strömungsreaktor verbundenen Mischkammer gemischt werden, vorzugsweise bei Raumtemperatur.

6. Verfahren nach Anspruch 1,
**dadurch gekennzeichnet, dass**
Ozon und NO₂ und/oder NO unter Verwendung eines Trägergases gemischt werden.

7. Verfahren nach Anspruch 1,
**dadurch gekennzeichnet, dass**
Ozon und NO₂ in einem Verhältnis eingesetzt werden, das kleiner 0,5 ist.

8. Verfahren nach Anspruch 1,
**dadurch gekennzeichnet, dass**
Ozon und NO in einem Verhältnis eingesetzt werden, das kleiner 1,5 ist.

9. Verfahren nach einem der Ansprüche 1 - 8,
**dadurch gekennzeichnet, dass**
es in einem Reaktor durchgeführt wird, der aus einem üblichen Strömungsreaktor (2) mit beheizbarem Außenmantel (3) und einer mit dem Strömungsreaktor (2) verbundenen Mischkammer (1) besteht, wobei an den Strömungsreaktor (2) gegebenenfalls ein GC-MS-Analysator und/oder eine Gasdurchflusszelle mit FT-IR-Spektrometer angeschlossen sind.

## Claims

1. A method of producing epoxides via oxidation of olefins in a homogeneous gas-phase reaction,
**characterized in that**
ozone and NO₂ and/or NO are mixed, the resulting gas mixture is fed into a flow reactor, and the olefin is reacted therein at a pressure of 0.1-1000 mbar and a temperature of 50-350°C.

2. The method according to claim 1,
**characterized in that**
the reaction is performed at a temperature of 100-300°C, preferably 140-240°C.

3. The method according to claim 1,
**characterized in that**
the reaction is performed at a pressure of 1-500 mbar, preferably 1-200 mbar.

4. The method according to claim 1,
**characterized in that**
monoolefins having 2 to 16 carbon atoms or diolefins having 4 to 16 carbon atoms are employed.

5. The method according to claim 1,
**characterized in that**
ozone and NO₂ and/or NO are mixed in a mixing chamber connected with the flow reactor, preferably at room temperature.

6. The method according to claim 1,
**characterized in that**
ozone and NO₂ and/or NO are mixed using a carrier gas.

7. The method according to claim 1,
**characterized in that**
ozone and NO₂ are employed at a ratio smaller than 0.5.

8. The method according to claim 1,
**characterized in that**
ozone and NO are employed at a ratio smaller than 1.5.

9. The method according to any of claims 1-8,
**characterized in that**
the method is carried out in a reactor comprised of a conventional flow reactor (2) with heatable exterior jacket (3) and a mixing chamber (1) connected with the flow reactor (2), the flow reactor (2) optionally being connected to a GC-MS analyzer and/or a gas flow cell with an FT-IR spectrometer.

## Revendications

1. Procédé de fabrication d'époxydes par oxydation d'oléfines dans une réaction homogène en phase gazeuse,
**caractérisé en ce**
**qu'**on mélange de l'ozone et du NO₂ et/ou du NO, qu'on introduit le mélange gazeux obtenu dans un réacteur à circulation et qu'on met en réaction ce mélange avec l'oléfine dans ce réacteur à une pression de 0,1 à 1000 mbars et à une température de 50 à 350°C.

2. Procédé selon la revendication 1,
**caractérisé en ce**
**qu'**on effectue la réaction à une température de 100 à 300°C, de préférence de 140 à 240°C.

3. Procédé selon la revendication 1,
**caractérisé en ce**
**qu'**on effectue la réaction à une pression de 1 à 500 mbars, de préférence de 1 à 200 mbars.

4. Procédé selon la revendication 1,
**caractérisé en ce**
**qu'**on utilise des monooléfines comportant de 2 à 16 atomes de carbone ou des dioléfines comportant de 4 à 16 atomes de carbone.

5. Procédé selon la revendication 1,
**caractérisé en ce**
**qu'**on mélange de l'ozone et du NO₂ et/ou du NO dans une chambre de mélange reliée au réacteur à circulation, de préférence à température ambiante.

6. Procédé selon la revendication 1,
**caractérisé en ce**
**qu'**on mélange de l'ozone et du NO₂ et/ou du NO en utilisant un gaz porteur.

7. Procédé selon la revendication 1,
**caractérisé en ce**
**qu'**on utilise de l'ozone et du NO₂ dans un rapport inférieur à 0,5.

8. Procédé selon la revendication 1,
**caractérisé en ce**
**qu'**on utilise de l'ozone et du NO dans un rapport inférieur à 1,5.

9. Procédé selon une des revendications de 1 à 8,
**caractérisé en ce**
**qu'**il est réalisé dans un réacteur composé d'un réacteur à circulation traditionnel (2) comportant une enveloppe extérieure chauffante (3) et d'une chambre de mélange (1) reliée au réacteur à circulation (2), et qu'éventuellement un analyseur GC-MS et/ou une cellule à circulation de gaz comportant un spectromètre FT-IR sont connectés dans le réacteur à circulation (2).
